# EUROPEAN PATENT SPECIFICATION

(11) **EP 0 458 543 B1**
(45) Date of publication and mention of the grant of the patent: **29.03.1995**
(21) Application number: 91304472.3
(22) Date of filing: 17.05.1991
(51) Int. Cl.: A61J 1/00, A61M 5/178

(54) **Suction transfer assembly**
Transfervorrichtung durch Saugen
Dispositif de transfert par aspiration

(30) Priority: 25.05.1990 US 529203
(43) Date of publication of application: 27.11.1991
(73) Proprietor: DBL INC., Scottsdale, Arizona (US)
(72) Inventor: Rohrbough, John, Scottsdale, Arizona 85259 (US)
(74) Representative: Moon, Donald Keith

(56) References cited:
- EP-A- 126 718
- EP-A- 417 988
- US-A- 3 563 373
- US-A- 4 180 070
- US-A- 4 535 820
- US-A- 4 768 568
- US-A- 4 834 149

## Description

The present invention relates to a suction transfer assembly for a medicament liquid, to an adapter for such an assembly and to a method of using such an assembly. An assembly according to the preamble of Claim 1 and an adapter according to the preamble of Claim 12 are known e.g. from US-A-4 180 070.

The present invention in particular relates to a suction transfer assembly of a vial having a chamber filled with a medicament liquid, such as a hazardous liquid, and closed by a stopper on which the vial is longitudinally slidable and a flow path containing adapter connectable to the vial by a hollow needle that penetrates the stopper, usable with a suction operating dispenser device, e.g. a syringe, lockably connectable to the adapter at a check valve in the flow path to transfer liquid from the vial to the device, with simultaneous compensating vial movement relative to the stopper under external atmospheric pressure to reduce the chamber volume by an amount equal to the liquid transferred, yet preventing leakage from the adapter on disconnecting the device.

Devices that hold and dispense hazardous materials, such as hazardous liquids in the medical field, are dangerous to use due to possible leakage from the device and contamination of the environment or user. Among such hazardous materials are cytotoxic or antineoplastic (oncology) drugs administered in chemotherapeutic treatment of cancer. They are usually stored as an injectable solution in a stoppered vial, prone to aerosol or droplet formation, as when manipulated at differential pressures in withdrawing a dosage into a syringe, since these operations change the saturated vapour pressure conditions of the volatilized constituents in the air space above the solution in the vial.

United States patent No. 4,180,070 to Genese describes a disposable syringe wherein one vial is received in the syringe body which also accommodates a double-pointed cannula having a hub member and a second vial is retained by an adapter which fits over the outside of the syringe barrel to provide upon movement of the adapter over the syringe barrel fluid communication between the two vials and intermixing of the two components. The cannula is positioned in the syringe barrel and has a hub with two frictional stop portions in conjunction with the syringe barrel.

United States patents 4,768,668 and 4,834,149 to Fournier et al show a hazardous material vial apparatus and conjoint operating method, in which the neck of a vial closed by a stationary stopper and containing a small amount of hazardous material powder and captive air is enclosed by a coaxial snap-on pressure release housing, formed as an open ended hollow cylinder whose outer end is closed by a septum and whose inner end openly faces the stopper, and which has an air pressure releasing hydrophobic filter vent near its outer end and a shiftable piston that divides the cylinder into an outer atmospheric chamber containing the vent and an inner pressure chamber openly facing the stopper.

According to these two patents, the needle of a syringe penetrates the septum, piston and stopper to add a small amount of liquid to the powder to form a hazardous solution and a saturated vapor thereof at increased pressure in the air space above the solution due to the added liquid volume. On inverting the arrangement, the increased pressure refills the syringe with the solution and returns the vial to atmospheric pressure. As the needle is removed, any escaping solution and aerosol formed from the vapor under the differential pressure conditions are retained in the inner chamber while the piston moves outwardly to adjust this chamber to atmospheric pressure, or are retained by the hydrophobic vent if they reach the outer chamber.

Alternatively, according to these patents, if the solution is not used immediately, the arrangement is kept upright, enough air is drawn into the syringe to return the vial to atmospheric pressure, and the needle removed as before, but with the air in the syringe being discharged into the outer chamber for safe exit into the atmosphere while any hazardous solution and aerosol are retained by the hydrophobic vent. When the solution is to be used, the syringe is loaded with a volume of air equal to that of the solution to be removed, this air is charged to the vial to increase its air pressure, the arrangement inverted, the solution volume drawn into the syringe and the vial returned to atmospheric pressure, and the needle removed as before.

It would thus be desirable to provide a suction transfer assembly for a medicament liquid, especially a hazardous liquid, that permits safe and easy transfer of the liquid from its container to a suction operating dispenser device such as a syringe, yet avoids leakage on detaching the device from the assembly.

It is accordingly an object of the present invention to overcome, or at least alleviate, one or more of the difficulties related to the prior art.

According to the invention, a suction transfer assembly for a medicament liquid, such as a hazardous liquid, is provided which comprises a longitudinal vial and a longitudinal adapter.

The vial forms an enclosed longitudinal chamber filled with the medicament liquid and has an open end closed by a penetrable stopper. The vial is slidable longitudinally relative to the stopper and the stopper slidably seals the liquid in the chamber in any position of vial sliding movement. Desirably, the liquid filled vial chamber is substantially free from air spaces. Also, the stopper has fastening means facing the vial open end.

The adapter has a front end, a rear end and an internal flow path extending therethrough. The rear end has a rearwardly facing hollow needle with a pointed tip defining the flow path rear terminus, and counterpart fastening means to the stopper fastening means. The rear end is insertable into the vial open end to fasten the adapter fastening means to those of the stopper to fasten the adapter to the stopper, and to penetrate the stopper by the needle to connect the chamber and flow path.

The adapter front end has a forwardly facing hollow nozzle defining the flow path front terminus, a mechanical contact probe-openable and self-closing check valve in the flow path adjacent the nozzle, and a lock connector, e.g. a luer lock, adapted to lock releasably to a counterpart lock connector, e.g. a counterpart luer lock, on a separate suction operated dispenser device, e.g. a syringe, having a counterpart hollow nozzle containing a mechanical contact probe to connect with the nozzle and open the valve.

Accordingly the present invention provides a suction transfer assembly for a medicament liquid, e.g. a hazardous liquid, permitting its repeated safe and easy transfer in selective dosage amounts from a container to a suction operating dispenser device without leakage on detaching the device each time from the assembly.

On fastening the adapter fastening means to the stopper fastening means and penetrating the stopper by the needle, and locking the device lock connector to the adapter lock connector and connecting the device nozzle with the adapter nozzle and mechanically opening the valve by the contact probe, suction operation of the device will cause liquid transfer from the chamber via the adapter to the device and simultaneous compensating vial movement longitudinally relative to the stopper under external atmospheric pressure to reduce the chamber volume by an amount generally equal to the liquid transferred.

The adapter rear end may have a socket containing the needle with its tip located inwardly from the rear end, and also containing its fastening means. The adapter and stopper fastening means may be coacting thread connection means. Coacting separation inhibiting means may be provided on the adapter and stopper to inhibit their separation once they are fastened.

The vial, stopper and adapter are suitably of generally cylindrical shape and coaxially arranged, with the needle located centrally in the adapter. The liquid filled vial closed by the stopper, and the adapter, may be in the form of a package kit.

The invention also contemplates a method of using the assembly for essentially leakage free transfer of a selective volume of the medicament from the vial chamber via the adapter to the separate suction operated device, comprising the steps of
(a) fastening the adapter fastening means to those of the stopper and penetrating the stopper by the needle to fasten the adapter to the stopper and flow connect the vial chamber and adapter flow path,
(b) locking the device lock connector to that of the adapter to flow connect the device nozzle with the adapter nozzle and mechanically open the adapter check valve by the device contact probe to flow the device with the adapter flow path,
(c) transferring a selective volume of liquid from the vial chamber into the device by suction operation of the device and simultaneously effecting compensating vial movement longitudinally relative to the stopper under external atmospheric pressure to reduce the chamber volume by an amount generally equal to the liquid transferred, and
(d) disconnecting the device from the adapter by unlocking their lock connectors with accompanying self-closing of the check valve to prevent liquid leakage from the adapter nozzle.

Steps (b), (c) and (d) may be repeated to transfer further selective volumes of liquid from the vial chamber to the device.

Other objects of the invention will become apparent from within the specification and accompanying drawings, in which:
Figure 1 is a schematic sectional view of a suction transfer assembly according to an embodiment of the invention, showing the stoppered vial and adapter in unassembled condition, and an associated syringe as suction operating dispensing device;
Figure 2 is a schematic view of the assembly and device of Figure 1 showing the parts connected to each other for suction transfer of liquid from the vial via the adapter to the syringe;
Figure 3 is a sectional view of an alternative embodiment of the assembly showing an adapter and check valve assembly for use with a stoppered vial and associated suction operating dispensing device;
Figure 4 shows a front end view of the assembly shown in Figure 3; and
Figure 5 shows a perspective view of the rear end of the adapter shown in Figure 3 and an associated penetrable stopper.

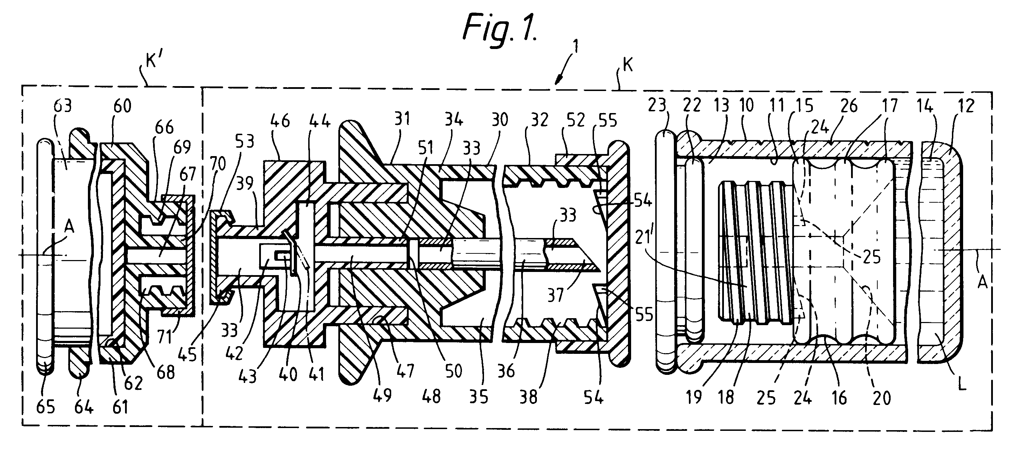

Referring to the drawings, and initially to Figure 1, a suction transfer assembly 1 is shown, including a vial 10 and an adapter 30, plus an associated suction operating or aspirating dispensing device in the form of a conventional syringe 60.

Vial 10 is shaped as a hollow longitudinal, e.g. glass, cylinder with a round cross sectional longitudinal inside wall 11 between its closed rear end 12 and open front end 13, to form an enclosed longitudinal chamber 14 filled with a medicament liquid L, such as a hazardous liquid, open end 13 being closed by a penetrable stopper 15. Vial 10 is slidable longitudinally relative to stopper 15 while stopper 15 slidably seals liquid L in chamber 14 in any position of vial sliding movement.

Stopper 15 is shaped as an elastomeric slide, e.g. rubber, cylinder with a round cross sectional body 16 having seal rings or ridges 17 slidably sealingly engaging vial wall 11 in the manner of a piston, and a round cross sectional extension 18 facing vial open end 13 and having fastening means formed by external threads 19, plus a central bore 20 closed by an internal penetrable seal 21 adjacent the front of extension 18.

Vial 10 is filled with liquid L to a maximum level yet sufficiently spaced inwardly from open end 13 to accommodate stopper 15 therein. Vial 10 is filled in a manner avoiding the presence of air spaces or pockets in chamber 14, to permit the liquid to undergo unhindered suction transfer or aspiration via stopper bore 20 on penetrating seal 21, as later described.

Vial 10 may have a bead 22 to engage a cap 23 on open end 13 to preserve vial 10 and stopper 15 in sterile condition.

The base of stopper extension 18 may have a circumferential formation of ratchets 24 and associated recesses 25 at the inner end of threads 19 for the purposed described below.

The volume of liquid L in chamber 14, in terms of the relative longitudinal position of vial 10 on stopper 15, is indicated by a series of indicia 26 along the barrel of vial 10. Liquid L may be standard hazardous material medicament solution, e.g. an injectable antineoplastic (oncology) solution.

Adapter 30 is shaped as a longitudinal, partially hollow, round cross sectional, e.g. rigid plastic, cylinder, with a front end 31 and rear end 32, and an internal flow path 33 extending through its body 34. Rear end 32 has a rearwardly opening socket 35 that contains a rearwardly facing hollow needle 36 with a pointed tip 37 defining the rear terminus of flow path 33, and counterpart fastening means formed by internal threads 38 that coact with external threads 19 on stopper 15. Rear end 32 is insertable into vial open end 13 and rotatable to fasten threads 38 to threads 19 to fasten adapter 30 to stopper 15, and simultaneously to cause needle 36 to enter stopper bore 20 and its tip 37 to penetrate seal 21 to connect chamber 14 and flow path 33.

Adapter front end 31 has a forwardly facing hollow nozzle 39 defining the front terminus of flow path 33. A mechanical contact probe-openable and self-closing check valve 40 is located in flow path 33 adjacent nozzle 39. Valve 40 is formed of a resilient diaphragm 40 having a forwardly facing hollow tubular nose 42 provided with liquid inflow apertures 43, and is situated in a valve chamber 44 with the edge portion of diaphragm 41 rigidly hingedly seated on the interior wall of chamber 44.

Nose 42 projects into the internal bore of nozzle 39 and diaphragm 41 seats sealingly against the adjacent wall of chamber 44 as valve seat at the entrance to that bore when valve 40 is in normally self-closed position, yet on inserting a mechanical contact probe into the bore of nozzle 39, nose 42 is contacted thereby and urged inwardly to lift diaphragm 41 from seated position to open valve (shown in phantom in Figure 1).

Nozzle 39 has a lock connector formed by an external luer lock 45, adapted to lock releasably to a counterpart lock connector, formed by an internal luer lock, on a separate suction operated dispenser device, e.g. syringe 60, having a counterpart hollow nozzle containing such a mechanical contact probe arranged to flow connect with nozzle 39 and open valve 40.

Nozzle 39, valve 40, diaphragm 41, nose 42, apertures 43, chamber 44 and luer lock 45, may be formed as a commercial (Burron Company) add-on unit 46 (cf. United States patent 4,535,820), mounted in an annular recess 47 in body 34 at front end 31 via its hollow annular chank 48, and with the bore 49 in its central extension 50 communicating with the inner end of needle 36 at interface 51 to provide this part of flow path 33 in adapter 30.

Adapter 30 may have a cap 52 on rear end 32 and a cap 53 on nozzle 39 to preserve it in sterile condition.

The outer edge of rear end 32 may have a circumferential counterpart formation of ratchets 54 and associated bosses 55 at the outer end of threads 38 in socket 35 arranged to coact with the formation of ratchets 24 and associated recesses 25 on stopper 15 for a locking grip between these parts when adapter 30 is rotated onto stopper 15 to achieve their interconnection and the penetrating of seal 21 by needle tip 37. As adapter threads 38 reach full rotational engagement with stopper threads 19, adapter ratchets 54 engage stopper ratchets 24 and adapter bosses 55 snap-lock in stopper recesses 25, respectively, such ratchets, bosses and recesses thus forming coacting separation inhibiting means to prevent separation of adapter 30 from stopper 15.

Syringe 60 is a conventional suction operated dispenser device of the type usable with assembly 1, having a hollow cylinder 61 whose chamber 62 slidably sealingly receives a piston plunger 63 for reciprocation by finger manipulation of cylinder flange 64 and plunger flange 65, plus a hollow nozzle 66 communicating via its central bore 67 with chamber 62 for aspiration or suction intake of fluid into chamber 62 on outward movement of plunger 63 from cylinder 61, e.g. when nozzle 66 is flow connected to adapter 30, and for pressure dispensing of fluid from chamber 62 to inward movement of plunger 63 into cylinder 61, e.g. when nozzle 66 is later flow connected to an injection needle (not shown) for administering liquid L to a patient.

For this purpose, nozzle 66 is formed as a counterpart nozzle that mates with adapter nozzle 39, and has an annular recess 68 coaxial to bore 67 and containing a counterpart lock connector formed by internal liner lock 69 that mates and releasably locks with adapter liner lock 45. Recess 68 is formed so that bore 67 outwardly ends in a tubular tip 70 defining a mechanical contact probe. Nozzle 66 may have a cap 71 to preserve syringe 60 in sterile condition.

Syringe nozzle 66 is connected to adapter nozzle 39 by rotating insertion of adapter luer lock 45 onto syringe liner lock 69 in recess 68 to interlock locks 45 and 69, thereby also flow connecting syringe bore 67 with adapter flow path 33 and mechanically opening check valve 40 as probe 70 urges valve nose 42 to unseat diaphragm 41 from its closed position to open position.

Assembly 1, formed of vial 10 filled with liquid L and closed by stopper 15, protected by end cap 23, and of adapter 30, protected by end caps 52 and 53, may be provided in unassembled sterile condition in a sterilely packaged kit K (shown in phantom in Figure 1) prepared under suitable safeguards and controlled conditions at the point of manufacture. Optionally, syringe 60, protected by end cap 71, may be included therewith as a composite sterilely packaged kit K' (shown in phantom in Figure 1).

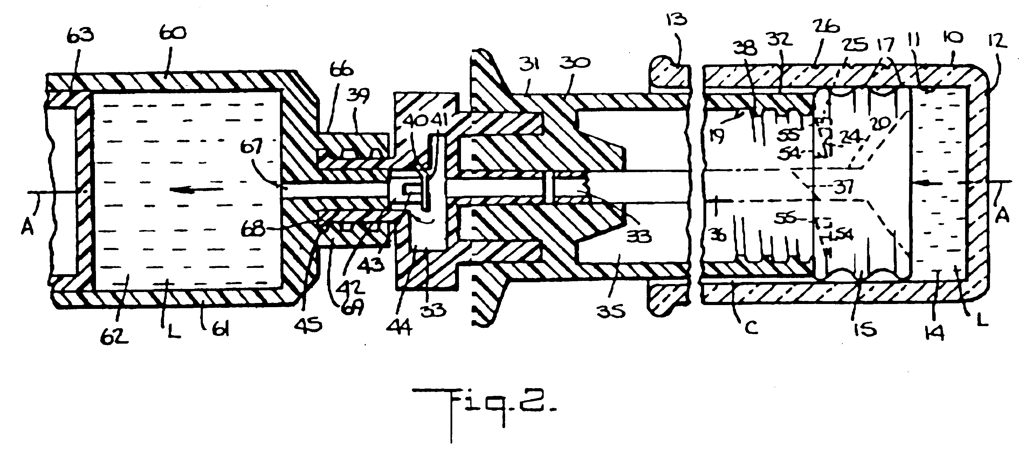

At the end point of use, e.g. on recovering the components from such a kit K or K', cap 23 is removed from vial open end 13 and cap 52 is removed from adapter rear end 32, rear end 32 is inserted into open end 13 and rotated to engage adapter threads 38 with stopper threads 19 and adapter ratchets 54 with stopper ratchets 24, to cause adapter needle 36 to enter stopper bore 20 and needle tip 37 to penetrate seal 21, and adapter bosses 55 to snap-lock in recesses 25, thereby generally permanently rigidly locking and flow connecting adapter 30 to stopper 15 (Figure 2).

Needle tip 37 is recessed in socket 35 at least slightly inwardly from rear end 32, so that adapter 30 may be safely manipulated to fasten it on stopper 15 without danger of needle tip 37 puncturing the user. Needle tip 37 is positioned relative to threads 38, whereby on fastening threads 38 to threads 19, needle tip 37 will penetrate seal 21 and protrude slightly therebeyond in bore 20 but will not enter chamber 14.

Adapter 30 is sized relative to vial inside wall 11 for insertion of rear end 32 into vial 10 with a slight clearance C therebetween, since adapter 30 is not connected to vial 10, but rather to stopper 15, and in permanent rigid manner. Thus, slight radial movement of adapter 30 relative to vial 10 may be accommodated, stopper 15 is desirably made of pliable or resilient, e.g. rubber, material, which insures a tight slidably sealing fit between its seal rings 17 and vial inside wall 11, in the manner of a piston-cylinder arrangement, yet permits adjustment between adapter 30 and vial 10 at clearance C.

Since valve 40 is a check valve, no leakage of liquid L can occur thereat on penetrating stopper seal 21 by adapter needle tip 37. Also, as adapter flow path 33 including chamber 44 and needle 37, and stopper bore 20, are collectively of very small volume, and as vial chamber 14 is free from air spaces and at an internal pressure equal to external atmospheric pressure, any air in flow path 33 and bore 20 is negligible and will not disturb the atmospheric pressure condition in chamber 14.

Syringe 60, e.g. on recovery from kit K′, may be connected to assembled vial 10, stopper 15 and adapter 30, by removing its end cap 71, inserting adapter nozzle 39 into recess 68 of syringe nozzle 66, and rotating these parts until adapter luer lock 45 is locked onto syringe luer lock 69 and syringe probe 70 is simultaneously inserted into adapter nozzle 39 to contact and shift valve nose 42 to raise diaphragm 41 to open position. Outward movement of plunger 63 from cylinder 61 aspirates a selective dosage of liquid L into syringe chamber 62 by suction withdrawal from vial chamber 14 via adapter flow path 33, passing outwardly around diaphragm 41 and inwardly through apertures 43 of nose 42 to reach syringe bore 67, as shown by the arrows in Figure 2.

At the same time, a vacuum is created in vial chamber 14, causing vial 10 to shift toward syringe 60 on stopper 15 and increasingly onto adapter 30 by simultaneous compensating movement longitudinally relative to stopper 15 under external atmospheric pressure that reduces the volume of chamber 14 by an amount equal to the volume of liquid L transferred to syringe 50. Vial chamber 14 always remains at an internal pressure generally equal to external atmospheric pressure and its reduced volume always remains free from air spaces. Vial 10 moves in unison with plunger 53 and stops immediately when plunger 63 stops.

The arrangement of vial 10, stopper 15, adapter 30 and syringe 60, and their pertinent parts, at the end point of use, is favourably coaxial relative to a common central axis A, yet since these components are of hand held size and used to administer comparatively small dosages of liquid L, the overall length of the interconnected components is relatively short (Figure 2). Advantageously, vial 10, stopper 15 and adapter 30 are of generally cylindrical shape and coaxially arranged, with needle 36 centrally located in adapter 30 in alignment with stopper bore 20 and adapter threads 39 in alignment with stopper threads 19.

Due to the maintaining of chamber 14 completely filled with liquid L and substantially free from air spaces, and the compensating volume reduction of chamber 14 as syringe 60 withdraws a selective liquid dosage therefrom, the interconnected arrangement of the components may be in any spatial orientation during liquid transfer, i.e. upright, inverted or at any angular position, without disturbing the transfer, as its internal system is at the same pressure as external atmospheric pressure, and formation of a saturated vapor of the liquid therein is prevented.

On disconnecting syringe nozzle 66 from adapter 39, cap 53 may be placed on nozzle 39 and a standard injection needle or other implement is mounted on nozzle 66 to administer the syringe dosage to the patient. As check valve 40 simultaneously self-closes on removing syringe probe 70 from contact with nose 42, leakage of liquid L from adapter nozzle 39 is safely prevented.

For the next dosage, the above operation is repeated, with the same or a different syringe 60 connected to adapter 30 to transfer from vial 10 an amount or liquid L corresponding to that dosage, resulting in the further shifting of vial 10 on stopper 15 by a compensating increment to reduce the volume of chamber 14 by a like amount to that of the liquid volume withdrawn.

A main advantage of the invention is that the hazardous liquid L is prepared, filled into vial 10 and closed by stopper 15, under controlled conditions at the point of manufacture which safeguard against leakage or other mishap and enable the liquid to be sealed by stopper 15 substantially free from attendant air spaces or pockets in chamber 14 that would foster a saturated hazardous vapor condition, lead to non-uniform pressure in chamber 14 on aspirating liquid dosages from vial 10 at the end point of use, and expose the environment and user to the danger of hazardous liquid leakage, e.g. in aerosol or droplet form.

Instead, on aspirating liquid L from vial 10, the internal pressure in chamber 14 always remains generally equal to external atmospheric pressure, and the concordant volume reduction of chamber 14 by compensating shifting of vial 10 on stopper 15 insures that chamber 14 will remain free of air spaces and at uniform internal pressure and thus inhibit aerosol or droplet formation of leakage from nozzle 39, which advantageously is further inhibited by the presence of check valve 40 thereat. As adapter 30 is desirably permanently fastened to stopper 15, nozzle 39 constitues the only leakage site of assembly 1, and leakage is avoided both by providing check valve 40 thereat and by automatically keeping chamber 14 on atmospheric pressure.

Forming adapter 30 as freely detachable from stopper 15, and stopper 15 as a self-sealing stopper are undesired, as this arrangement increases the possibility of leakage at the end point of use. It would also leave exposed needle tip 37 wet with residual liquid L, posing the danger of contaminating the environment or wetting or puncturing the skin of the user, even if cap 52 were remounted on adapter rear end 32, as this would contaminate the cap and repeat the danger of user contact with residual liquid L thereat on removing the cap to reconnect the adapter to the stopper to withdraw the next dosage from vial 10.

Use of a self-sealing stopper in nozzle 39 for penetration by a syringe needle, instead of check valve 40 and probe 70 containing nozzle 66, is also undesired, for like reasons.

Instead of mixing materials to form a hazardous solution at the end point of use where the conditions cannot be adequately controlled, e.g. per the aforesaid two Fournier et al patents, according to the invention the medicament is provided as a liquid formulated at the point of manufacture and the manipulations needed to charge the syringe at the end point of use are minimized as is the danger to the environment and user.

Although standard luer lock connectors as embodied by mating luer locks 45 and 69 are preferred, any suitable lock formation may be employed to interlock nozzles 39 and 69 to provide a sealed flow connection between adapter 30 and syringe 60.

Vial 10, stopper 15 and adapter 30 may be provided as disposable items, i.e. to be discarded as a still assembled unit per an authorized disposal procedure for hazardous materials, when the supply of liquid L in chamber 14 has been essentially completely withdrawn by the above described transfer operation.

The suction transfer of hazardous liquid contemplated by assembly 1 is safer than with conventional systems, as transfer is achieved in a completely closed arrangement, in which from start to finish the user is never exposed to an unprotected needle, nor to danger of leakage on disconnecting the syringe from the adapter, as the adapter has a check valve, and the liquid is always at atmospheric pressure in the assembly and syringe and will not spray forth from either disconnected nozzle. Since the disconnected syringe is needle-less it poses no danger of puncturing the user with a hazardous liquid containing needle.

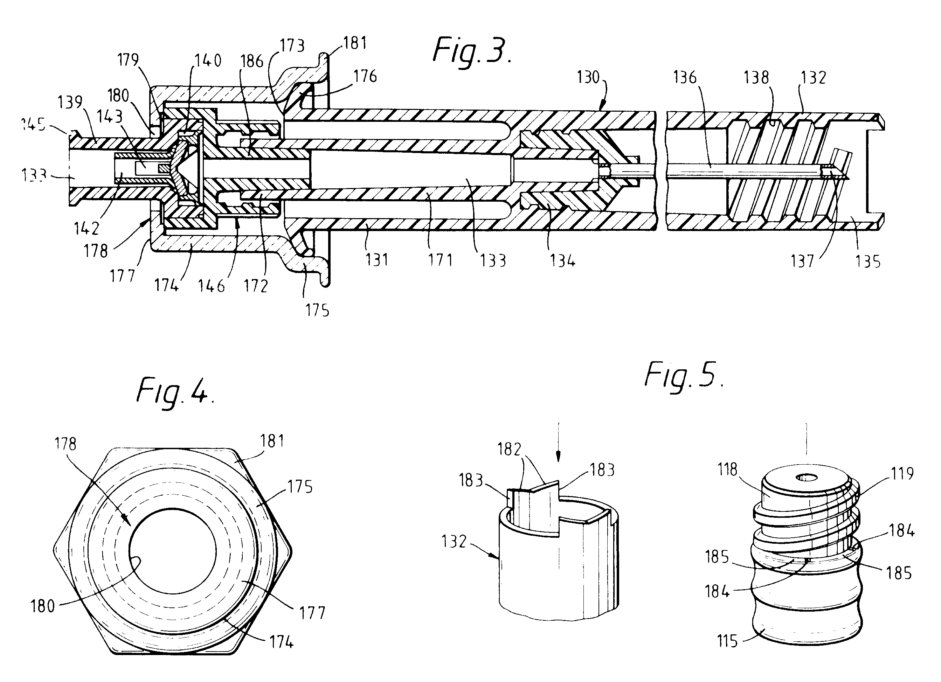

Figures 3 to 5 of the drawings depict an embodiment of the invention which is similar to that described above but which has certain additional features as discussed below. Elements of the assembly depicted in Figures 3 to 5 which are similar to those shown in Figures 1 and 2 have been numbered with the same numerals as the elements in Figures 1 and 2 except with a "1" prefix before the number so all elements in Figures 3 to 5 are numbered in the range 100 to 199.

As shown, an adapter 130 is adapted to be used with a vial and syringe of the type described hereinbefore but not shown in these drawings for the sake of clarity. A front part of adapter 130 is of cylindrical shape and has a front end 131 and a rear end 132. Rear end 132 has a rearwardly opening socket 135 that contains a rearwardly facing hollow needle 136 with a pointed tip 137 defining the rear terminus of a flow portion 133. Counterpart fastening means in the form of internal threads 138 are adapted to coact with the external threads 119 of a vial stopper 115 in the manner described hereinbefore.

The front end 131 of the front part of the adapter 130 has a forwardly facing hollow nozzle 139 defining the front terminus of flow portion 133. A check valve unit 146 forms a second part of the adapter 130 and is connected to the body 134 at the front end 131, and nozzle 139 forms part of unit 146. Adapter body 134 has a forwardly facing hollow hub 171 which has a forward facing end 172 which stands proud of the forward facing end face 173 of the body 134. Hub 171 forms a part of flow path 133. Unit 146 has a rearwardly facing tapered nozzle 186 which fits into hub 171 in a fluid tight manner so that no fluid leakage occurs between unit 146 and hub 171 during use of the adapter.

A cap 174 fits over unit 146 to hold unit 146 tightly to body 134. Cap 174 preferably includes a skirt 175 which locates over an outwardly directed flange 176 on body 134. Cap 174 has an inwardly directed lip 177 at its forward end 178 lip 177 engaging a shoulder 179 on unit 146 to securely hold unit 146 to body 134. A central aperture 180 in the forward end 178 of cap 174 allows nozzle 139 to project through cap 174 beyond end face 178. Nozzle 139 has a luer lock connection 145 on the end thereof. Unit 146 may be a commercial unit of the type manufactured by Burron Company (c.f. United States Patent 4,535,820).

Cap 174 may have an outwardly directed flange 181 on the free end of skirt 175. Flange 181 is preferably of non-circular form and may be hexagonal as depicted clearly in Figure 4 of the drawings. Flange 181 facilitates separation of unit 146 from the body 134, and the non-circular form thereof prevents the adapter and the vial assembly rolling freely on an inclined surface. Since the vial will generally be of circular cross-sectional form it would, in the absence if flange 181, roll freely on an even slightly inclined surface, which is particularly dangerous should the vial contain hazardous medicaments and be inadvertently permitted to roll onto the floor and shatter. Flange 181 of non-circular form prevents this occurring when the adapter and vial are in assembly.

The rear end 132 of body 134 may have at least one pair, and preferably two pairs of counteracting ratchets 182 with associated bosses 183 thereon. The bosses 183 face away from each other so that a pair of "bow tie" shaped formations are formed on the end 132 of body 134. The stopper 115 preferably has four radially extending projections 184 at the base of the stopper extension 118 and associated recesses 185. When adapter threads 138 reach full rotational engagement with stopper thread 119 adapter ratchets 182 ride over projections 184 until adapter bosses 183 snap-lock in stopper recesses 185. Further relative rotation of stopper 115 and adapter 130 in either direction is thus inhibited by the bosses 183 coacting with the projections 184 on opposite sides of the recesses 185. Thus coaction is adapted to prevent adapter 130 screwing too far into the stopper 115 and in so doing damaging the stopper 115. This coaction also prevents stopper 115 and adapter 130 inadvertently screwing apart.

An advantage of holding the valve unit 146 to the nose 134 with a cap 174 is that no gluing or other permanent fixing operation is required to effect the assembly of the adapter.

The specification and drawings are set forth by way of illustration and not limitation, and various modifications may be made therein without departing from the invention which is to be limited solely by the scope of the claims.

## Claims

1. A suction transfer assembly (1) for a medicament liquid (L) comprising a vial (10) and adapter (30), the vial forming an enclosed longitudinal chamber (14) filled with a medicament liquid and having an open end (13) closed by a penetrable stopper (15), the vial being slidable longitudinally relative to the stopper and the stopper slidably sealing the liquid in the chamber in any position of vial sliding movement, the stopper having fastening means (19) facing the vial open end (13), and
the adapter being a longitudinal adapter (30) having a front end (31) a rear end (32) having counterpart fastening means (38) to fasten the adapter to the fastening means (19) of the stopper, characterised in that the adpater has an internal flow path (33) extending therethrough, the rear end of the adapter having a rearwardly facing hollow needle (36) with a pointed tip (37) defining the rear terminus of the flow path, the rear end being insertable into the vial open end to fasten the counterpart fastening means to the fastening means for fastening the adapter to the stopper, and to penetrate the stopper by the needle for flow connecting the chamber and flow path, and
the front end having a forwardly facing hollow nozzle (39) defining the front terminus of the flow path, a mechanical contact probe-openable and self-closing check valve (40) in the flow path adjacent the nozzle and a lock connector (45) adapted to lock releasably to a counterpart lock connector (69) on a separate suction operated dispenser device (60) having a counterpart hollow nozzle (66) containing a mechanical contact probe (70) arranged for flow connection with the nozzle and for opening the valve,
whereby on fastening the counterpart fastening means (38) to the fastening means (19) and penetrating the stopper (18) by the needle (36), and locking the counterpart lock connector (69) to the lock connector (45) and flow connecting the counterpart nozzle (66) with the nozzle (39) and mechanically opening the valve by the contact probe, suction operation of the device (60) will cause liquid transfer from the chamber via the adapter (30) to the device (60) and simultaneously compensating vial movement longitudinally relative to the stopper under external atmospheric pressure to reduce the chamber volume by an amount generally equal to the liquid transferred.

2. An assembly according to claim 1 wherein the rear end (32) has a socket (35) containing the needle (36) and the needle tip (37) is located in the socket inwardly from the rear end.

3. An assembly according to claim 2 wherein the counterpart fastening means (38) are located in the socket (35).

4. An assembly according to any preceding claim wherein the counterpart fastening means (38) and fastening means (19) are coacting thread connection means.

5. An assembly according to any preceding claim wherein coacting separation inhibiting means (54,55) are provided on the adapter (30) and stopper (15) to inhibit separation of the counterpart fastening means (38) from the fastening means (19) after fastening thereof.

6. An assembly according to any preceding claim wherein the lock connector is a luer lock connector.

7. An assembly according to any preceding claim wherein the vial (10), stopper (15) and adapter (30) are of generally cylindrical shape and coaxially arranged with the needle (36) centrally located in the adapter.

8. An assembly according to any preceding claim wherein the liquid filled chamber (14) is substantially free from attendant air spaces.

9. An assembly according to any preceding claim wherein the liquid filled vial (10) closed by the stopper (15), and the adapter (30), are in unfastened condition in the form of a sterile packaged kit.

10. An assembly according to any preceding claim including a separate suction operated dispenser device (60) having a counterpart lock connector (69) for locking releasably to the lock connector (45) and a counterpart hollow nozzle (66) containing a mechanical contact probe (70) arranged for flow connection with the nozzle (39) and for opening the valve (40).

11. An assembly according to claim 10 wherein the suction operated suction device (60) is a syringe.

12. A longitudinal adapter (30) for a suction transfer assembly for transferring a medicament liquid from a vial closed by a penetrable stopper to a suction operated dispenser device the adapter having a front end (31) and a rear end (32) having counterpart fastening means (38), the rear end being adapted to be inserted into the open end of a liquid filled vial closed by a penetrable stopper (15) having fastening means (19) facing the vial open end (13), to fasten the counterpart fastening means to the fastening means for fastening the adapter to the stopper, characterized by an internal flow path (33) extending through said adapter,
the rear end having a rearwardly facing hollow needle (36) with a pointed tip (37) defining the rear terminus of the flow path and being adapted to penetrate the stopper by the needle for flow connecting the vial liquid and flow path, and
the front end having a forwardly facing hollow nozzle (39) defining the front terminus of the flow path, a mechanical contact probe-openable and self-closing check valve (40) in the flow path adjacent the nozzle, and a lock connector (45) adapted to lock releasably to a counterpart lock connector (69) on a separate suction operated dispenser device (60) having a counterpart hollow nozzle (66) containing a mechanical contact probe (70) arranged for flow connection with the nozzle and for opening the valve.

13. An adapter according to claim 12 wherein the rear end (32) has a socket (35) containing the needle (36) and the needle tip (37) is located in the socket inwardly from the rear end.

14. An adapter according to claim 13 wherein the counterpart fastening means (38) are located in the socket.

15. An adapter according to claim 14 wherein the counterpart fastening means (38) are thread connection means.

16. An adapter according to any of claims 12 to 15 wherein the lock connector is a luer lock connector.

17. An adapter (30) according to any of claims 12 to 16 wherein the adapter is of generally cylindrical shape with the needle (36) centrally located therein.

18. A method of using the assembly (1) according to claim 10 for essentially leakage free transfer of a selective volume of the medicament liquid (1) from the vial chamber (14) via the adapter (30) to the separate suction operated device (60), comprising the steps of
(a) fastening the counterpart fastening means (38) to the fastening means (19) and penetrating the stopper (15) by the needle (36) to fasten the adapter to the stopper and flow connect the vial chamber and adapter flow path (33),
(b) locking the counterpart lock connector (69) to the lock connector (45) to flow connect the counterpart nozzle (66) with the nozzle (38) and mechanically open the valve (40) by the contact probe (70) to flow connect the device with the adapter flow path,
(c) transferring a selected volume of the liquid from the chamber into the device by suction operation of the device and simultaneously effecting compensating vial movement longitudinally relative to the stopper under external atmospheric pressure to reduce the chamber volume by an amount generally equal to the liquid transferred, and
(d) disconnecting the device from the adapter by unlocking the counterpart lock connector from the lock connector with accompanying self-closing of the valve to prevent leakage from the nozzle.

19. A method according to claim 18 wherein the liquid filled chamber (14) is substantially free from attendant air spaces.

20. A method according to claim 18 or 19 wherein steps (b), (c) and (d) are repeated to transfer a further selective volume of the liquid from the vial chamber (14) to the device (60).

21. A method according to claim 18, 19 or 20 wherein the device (60) is a syringe.

22. An assembly according to any of claims 1 to 9 wherein the adapter (130) is formed of first and second parts, the valve (140) is housed within a unit (46) which forms said second part of the adapter, said unit being releasably mounted to the first part of the adapter.

23. An assembly according to claim 22 wherein said unit (146) is releasably held to said first part of the adapter by a cap (174), an opening (180) being formed in the cap through which the forward facing hollow nozzle (139) extends.

24. An assembly according to claim 23 wherein the cap (174) snap fits onto the first part of the adapter.

25. An assembly according to claim 24 wherein the cap (174) has an outwardly directed flange (181) which extends radially beyond the outer periphery of the vial (10) when the vial and adapter are in operative assembly, said cap flange being of non-circular configuration.

26. An adapter according to any one of claims 12 to 17 wherein the adapter is formed of first and second parts, the valve (140) being housed within a unit (146) which forms said second part, said unit being releasably mounted to the first part.

27. An adapter (130) according to claim 26 wherein said first part and second part are releasably held together by a cap (174), an opening (180) being formed in the cap through which the forward facing hollow nozzle (139) extends.

28. An adapter (130) according to claim 27 wherein said cap (174) snap fits onto said first part.

## Patentansprüche

1. Saugtransfervorrichtung (1) für eine medikamentöse Flüssigkeit (L) mit einem Behälter (10) und einem Adapter (30), wobei der Behälter eine mit einer medikamentösen Flüssigkeit gefüllte umschlossene längliche Kammer (14) mit einem durch einen durchlässigen Pfropfen (15) verschlossenen offenen Ende (13) bildet, und wobei der Behälter zum Propfen hin längsverschiebbar ist und der Pfropfen die Flüssigkeit in der Kammer bei jeder Schiebebewegung des Behälters durch Verschieben verschließt, und der Pfropfen Befestigungsmittel (19) zum offenen Ende (13) des Behälters hin aufweist, und
der Adapter ein länglicher Adapter (30) mit einem vorderen Ende (31) und einem hinteren Ende (32) ist, mit Gegenbefestigungsmitteln (38) zur Befestigung des Adapters an den Befestigungsmitteln (19) des Propfens, dadurch gekennzeichnet, daß der Adapter einen durch ihn verlaufenden internen Durchflußweg (33) aufweist, wobei das hintere Ende des Adapters eine nach hinten weisende hohle Nadel (36) mit einem den hinteren Endpunkt des Durchflußweges definierenden spitzen Ende (37) aufweist und das hintere Ende in das offene Ende des Behälters einsetzbar ist, um die Gegenbefestigungsmittel an den Befestigungsmitteln zur Befestigung des Adapters am Pfropfen zu befestigen und zum Eindringen der Nadel in den Pfropfen zur Fließverbindung der Kammer und des Durchflußweges, und
das vordere Ende eine den vorderen Endpunkt des Durchflußweges definierende nach vorne weisende hohle Kanüle (39) aufweist, ein an der Kanüle anliegendes den mechanischen Kontaktfühler öffnendes und selbstschließendes Absperrventil (40) im Durchflußweg sowie eine Verschlußverbindung (45) zum lösbaren Verschließen mit einer Gegenverschlußverbindung (69) an einer separaten durch Saugen betätigten Dispensorvorrichtung (60) mit einer Gegenhohlkanüle (66) mit einem mechanischen Kontaktfühler (70) zur Fließverbindung mit der Kanüle und zum Öffnen des Ventils,
wobei durch die Befestigung der Gegenbefestigungsmittel (38) an den Befestigungsmitteln (19) und durch das Eindringen der Nadel (36) in den Pfropfen (15) und durch Schließen der Gegenverschlußverbindung (69) mit der Verschlußverbindung (45) und durch die Fließverbindung der Gegenkanüle (66) mit der Kanüle (39) sowie durch das mechanische Öffnen des Ventils durch den Kontaktfühler ein Saugvorgang der Vorrichtung (60) den Flüssigkeitstransfer von der Kammer über den Adapter (30) zur Vorrichtung (60) auslöst und gleichzeitig eine Längsbewegung des Behälters zum Pfropfen hin unter externem atomsphärischem Druck ausgleicht, um das Volumen in der Kammer um eine im allgemeinen der transferierten Flüssigkeit entsprechenden Menge zu reduzieren.

2. Vorrichtung nach Anspruch 1, worin das hintere Ende (32) eine die Nadel (36) enthaltende Fassung (35) aufweist, und die Nadelspitze (37) in der Fassung vom hinteren Ende nach innen angebracht ist.

3. Vorrichtung nach Anspruch 2, worin die Gegenbefestigungsmittel (38) in der Fassung (35) angebracht sind.

4. Vorrichtung nach einem der vorstehenden Ansprüche, worin die Gegenbefestigungsmittel (38) und die Befestigungsmittel (19) zusammenwirkende Gewindeverbindungsmittel sind.

5. Vorrichtung nach einem der vorstehenden Ansprüche, worin zusammenwirkende trennhemmende Mittel (54, 55) am Adapter (30) sowie am Pfropfen (15) angebracht sind, um die Trennung der Gegenbefestigungsmittel (38) von den Befestigungsmitteln (19) nach deren Befestigung zu verhindern.

6. Vorrichtung nach einem der vorstehenden Ansprüche, worin die Verschlußverbindung eine Luer-Verschlußverbindung ist.

7. Vorrichtung nach einem der vorstehenden Ansprüche, worin der Behälter (10), der Pfropfen (15) und der Adapter (30) im allgemeinen zylinderförmig und mit der im Adapter zentral angeordneten Nadel (36) koaxial angeordnet sind.

8. Vorrichtung nach einem der vorstehenden Ansprüche, worin die mit der Flüssigkeit gefüllte Kammer (14) im wesentlichen frei von damit verbundenen Lufträumen ist.

9. Vorrichtung nach einem der vorstehenden Ansprüche, worin der mit Flüssigkeit gefüllte vom Pfropfen (15) verschlossene Behälter (10) und der Adapter (30) im unbefestigten Zustand in Form eines sterilen Bausatzes vorliegen.

10. Vorrichtung nach einem der vorstehenden Ansprüche, mit einer separaten durch Saugen betätigten Dispensorvorrichtung (60), die eine Gegenverschlußverbindung (69) zum lösbaren Verschließen mit der Verschlußverbindung (45) sowie eine Gegenhohlkanüle (66) mit einem mechanischen Kontaktfühler (70) aufweist, zur Fließverbindung mit der Kanüle (39) und zum Öffnen des Ventils (40).

11. Vorrichtung nach Anspruch 10, worin die durch Saugen betätigte Saugvorrichtung (60) eine Spritze ist.

12. Ein länglicher Adapter (30) für eine Saugtransfervorrichtung, zum Transferieren einer medikamentösen Flüssigkeit von einem mit einem durchdringbaren Pfropfen verschlossenen Behälter in eine durch Saugen betätigte Dispensorvorrichtung, wobei der Adapter ein vorderes Ende (31) und ein hinteres Ende (32) mit Gegenbefestigungsmitteln (38) aufweist, wobei das hintere Ende derart ist, daß es in das offene Ende einer mit Flüssigkeit gefüllten von einem durchdringbaren Pfropfen (15) geschlossenen Behälter eingesetzt werden kann, mit zum offenen Ende (13) des Behälters weisenden Befestigungsmitteln (19), zur Befestigung der Gegenbefestigungsmittel an den Befestigungsmitteln zum Befestigen des Adapters an dem Pfropfen, worin ein interner Durchflußweg (33) durch den Adapter verläuft,
wobei das hintere Ende eine nach hinten weisende hohle Nadel (36) mit einem den Endpunkt des Durchflußweges definierenden spitzen Ende (37) aufweist, die derart ist, daß sie den Pfropfen zur Fließverbindung der Behälterflüssigkeit und des Durchflußweges durchdringt, und
wobei das vordere Ende eine den vorderen Endpunkt des Durchflußweges definierende nach vorne weisende hohle Kanüle (39), ein an der Kanüle anliegendes, einen mechanischen Kontaktfühler öffnendes und selbstschließendes Absperrventil (40) im Durchflußweg sowie eine Verschlußverbindung (45) zum lösbaren Verschließen mit einer Gegenverschlußverbindung (69) an einer separaten durch Saugen betätigten Dispensorvorrichtung (60) mit einer Gegenhohlkanüle (66) mit einem mechanischen Kontaktfühler (70) zur Fließverbindung mit der Kanüle und zum Öffnen des Ventils aufweist.

13. Adapter nach Anspruch 12, worin das hintere Ende (32) eine Fassung (35) mit einer Nadel (36) aufweist, und die Nadelspitze (37) in der Fassung vom hinteren Ende nach innen angebracht ist.

14. Adapter nach Anspruch 13, worin die Gegenbefestigungsmittel (38) in der Fassung angebracht sind.

15. Adapter nach Anspruch 14, worin die Gegenbefestigungsmittel (38) Gewindeverbindungsmittel sind.

16. Adapter nach einem der Ansprüche 12 bis 15, worin die Verschlußverbindung eine Luer-Verschlußverbindung ist.

17. Adapter (30) nach einem der Ansprüche 12 bis 16, worin der Adapter allgemein zylinderförmig mit der zentral darin angeordneten Nadel (36) ist.

18. Verfahren zur Verwendung der Vorrichtung (1) nach Anspruch 10 zum im wesentlichen leckfreien Transfer eines selektiven Teils der medikamentösen Flüssigkeit (L) aus der Behälterkammer (14) über den Adapter (30) zur separaten durch Saugen betätigten Vorrichtung (60), worin folgende Schritte enthalten sind:
(a) Befestigen der Gegenbefestigungsmittel (38) an den Befestigungsmitteln (19) und Eindringen der Nadel (36) in den Pfropfen (15) zur Befestigung des Adapters am Pfropfen und zur Fließverbindung der Behälterkammer und des Durchflußweges (33) des Adapters,
(b) Verschließen der Gegenverschlußverbindung (69) mit der Verschlußverbindung (45) zur Fließverbindung der Gegenkanüle (66) mit der Kanüle (39) und zum mechanischen Öffnen des Ventils (40) durch den Kontaktfühler (70) zur Fließverbindung der Vorrichtung mit dem Durchflußweges des Adapters,
(c) Transferieren eines ausgewählten Teiles der Flüssigkeit von der Kammer in die Vorrichtung durch Saugbetätigung der Vorrichtung und gleichzeitiges Ausgleichen der Längsbewegung des Behälters zum Pfropfen hin unter externem atomsphärischem Druck, um das Volumen in der Kammer um eine im allgemeinen der tranferierten Flüssigkeit entsprechenden Menge zu reduzieren,
(d) Trennen der Vorrichtung vom Adapter durch Lösen der Gegenverschlußverbindung von der Verschlußverbindung bei gleichzeitigem selbsttätigem Schließen des Ventils zur Vermeidung von Lecken der Kanüle.

19. Verfahren nach Anspruch 18, worin die mit Flüssigkeit gefüllte Kammer (14) im wesentlichen frei von damit verbundenen Lufträumen ist.

20. Verfahren nach Anspruch 18 oder 19, worin Schritte (b), (c) und (d) zum Transfer eines weiteren selektiven Flüssigkeitteiles von der Behälterkammer (14) zur Vorrichtung (60) wiederholt werden.

21. Verfahren nach Anspruch 18, 19 oder 20, worin die Vorrichtung (60) eine Spritze ist.

22. Vorrichtung nach einem der Ansprüche 1 bis 9, worin der Adapter (130) aus ersten und zweiten Teilen gebildet ist, das Ventil (140) in einer den zweiten Teil des Adapters bildenden Einheit (146) angebracht ist, wobei die Einheit lösbar am ersten Teil des Adapters angebracht ist.

23. Vorrichtung nach Anspruch 22, worin die Einheit (146) durch eine Kappe (174) lösbar am ersten Teil des Adapters gehalten wird, wobei die nach vorn weisende hohle Kanüle (139) durch die in der Kappe gebildete Öffnung (180) verläuft.

24. Vorrichtung nach Anspruch 23, worin die Kappe (174) einrastend auf dem ersten Teil des Adapters angebracht ist.

25. Vorrichtung nach Anspruch 24, worin die Kappe (174) einen nach außen gerichteten Flansch (181) aufweist, der bei Funktionsstellung des Behälters und des Adapters radial über die äußere Peripherie des Behälters (10) verläuft, wobei der Flansch unrund ausgebildet ist.

26. Adapter nach einem der Ansprüche 12 bis 17, worin der Adapter aus ersten und zweiten Teilen gebildet ist und das Ventil (140) innerhalb einer den zweiten Teil bildenden Einheit (146) angeordnet ist, und die Einheit lösbar auf dem ersten Teil angebracht ist.

27. Adapter (130) nach Anspruch 26, worin der erste und der zweite Teil lösbar durch eine Kappe (174) zusammengehalten werden, wobei die nach vorn weisende hohle Kanüle (139) durch eine in der Kappe gebildete Öffnung (180) verläuft.

28. Adapter (130) nach Anspruch 27, worin die Kappe (174) einrastend auf dem ersten Teil angebracht ist.

## Revendications

1. Ensemble (1) de transfert par aspiration de liquide médicamenteux (L) comportant un flacon (10) et un adaptateur (30), le flacon formant une chambre longitudinale fermée (14) remplie d'un liquide médicamenteux et ayant une extrémité ouverte (13) fermée par un dispositif d'arrêt (15) pouvant être percé, le flacon pouvant coulisser longitudinalement par rapport au dispositif d'arrêt et le dispositif d'arrêt assurant de manière coulissante l'étanchéité du liquide situé dans la chambre dans toute position du mouvement coulissant du flacon, le dispositif d'arrêt ayant des moyens de fixation (19) dirigés vers l'extrémité ouverte (13) du flacon, et l'adaptateur étant un adaptateur longitudinal (30) ayant une extrémité avant (31), une extrémité arrière (32) comportant des moyens de fixation complémentaires (38) destinés à fixer l'adaptateur sur les moyens de fixation (19) du dispositif d'arrêt, caractérisé en ce que l'adaptateur a un trajet d'écoulement intérieur (33) s'étendant à travers celui-ci, l'extrémité arrière de l'adaptateur ayant une aiguille creuse (36) dirigée vers l'arrière ayant un bout pointu (37) définissant l'extrémité arrière du trajet d'écoulement, l'extremité arrière pouvant être insérée à l'intérieur de l'extrémité ouverte du flacon pour fixer les moyens de fixation complémentaires sur les moyens de fixation pour fixer l'adaptateur sur le dispositif d'arrêt, et pour percer le dispositif d'arrêt par l'intermédiaire de l'aiguille pour réaliser un écoulement reliant la chambre et le trajet d'écoulement, et l'extrémité avant ayant une buse creuse (39) dirigée vers l'avant définissant l'extrémité avant du trajet d'écoulement, un clapet anti-retour (40) pouvant être ouvert par une sonde de contact mécanique et se fermant par lui-même situé dans le trajet d'écoulement en étant adjacent à la buse et un raccord (45) formant verrou adapté pour être verrouillé de manière libérable sur un connecteur (69) formant verrou complémentaire situé sur un dispositif dispensateur séparé (60) actionné par aspiration comportant une buse creuse complémentaire (66) contenant une sonde de contact mécanique (70) agencée pour établir une liaison d'écoulement avec la buse et pour ouvrir le clapet, de sorte qu'en fixant les moyens de fixation complémentaires (38) sur les moyens de fixation (19) et en perçant le dispositif d'arrêt (18) à l'aide de l'aiguille (36), et en verrouillant le connecteur (69) formant verrou complémentaire sur le connecteur (45) formant verrou et en reliant pour écoulement la buse complémentaire (66) à la buse (39) et en ouvrant mécaniquement le clapet par la sonde de contact, l'opération d'aspiration du dispositif (60) entraîne un transfert de liquide depuis la chambre via l'adaptateur (30) vers le dispositif (60) et de manière simultanée la compensation du déplacement du flacon longitudinalement par rapport au dispositif d'arrêt soumis à la pression atmosphérique extérieure pour réduire le volume de la chambre d'une quantité de manière générale égale à celle du liquide transféré.

2. Ensemble selon la revendication 1, dans lequel l'extrémité arrière (32) comporte une tubulure (35) contenant l'aiguille (36) et le bout (37) de l'aiguille est situé dans la tubulure en direction de l'intérieur à partir de l'extrémité arrière.

3. Ensemble selon la revendication 2, dans lequel les moyens de fixation complémentaires (38) sont situés dans la partie tubulaire (35).

4. Ensemble selon l'une quelconque des revendications précédentes, dans lequel les moyens de fixation complémentaires (38) et les moyens de fixation (19) coopèrent à l'aide de moyens de liaison filetés.

5. Ensemble selon l'une quelconque des revendications précédentes, dans lequel des moyens complémentaires (54, 55) empêchant la séparation sont agencés sur l'adaptateur (30) et le dispositif d'arrêt (15) pour empêcher la séparation des moyens de fixation complémentaires (38) des moyens de fixation (19) après leur fixation.

6. Ensemble selon l'une quelconque des revendications précédentes, dans lequel le connecteur formant verrou est de type Luer.

7. Ensemble selon l'une quelconque des revendications précédentes, dans lequel le flacon (10), le dispositif d'arrêt (15) et l'adaptateur (30) ont une forme de manière générale cylindrique et sont agencés coaxialement à l'aiguille (36) située axialement dans l'adaptateur.

8. Ensemble selon l'une quelconque des revendications précédentes, dans lequel la chambre (14) remplie de liquide est pratiquement exempte d'espaces d'air d'accompagnement.

9. Ensemble selon l'une quelconque des revendications précédentes, dans lequel le flacon (10) rempli de liquide, fermé par le dispositif d'arrêt (15), et l'adaptateur (30), sont, dans l'état non-fixé, sous la forme d'un nécessaire emballé stérile.

10. Ensemble selon l'une quelconque des revendications précédentes, comportant un dispositif dispensateur (60) séparé actionné par aspiration ayant un connecteur (69) formant verrou complémentaire destiné à être verrouillé de manière libérable sur le connecteur (45) formant verrou et une buse creuse complémentaire (66) contenant une sonde de contact mécanique (70) agencée pour établir un écoulement avec la buse (39) et pour ouvrir le clapet (40).

11. Ensemble selon la revendication 10, dans lequel le dispositif dispensateur (60) actionné par aspiration est une seringue.

12. Adaptateur longitudinal (30) pour ensemble de transfert par aspiration destiné à transférer un liquide médicamenteux à partir d'un flacon fermé par un dispositif d'arrêt pouvant être percé vers un dispositif dispensateur actionné par aspiration, l'adaptateur ayant une extrémité avant (31) et une extrémité arrière (32) comportant des moyens de fixation complémentaires (38), l'extrémité arrière étant adaptée pour être insérée dans l'extrémité ouverte d'un flacon rempli de liquide et fermé par un dispositif d'arrêt (15) pouvant être percé comportant des moyens de fixation (19) dirigés vers l'extrémité ouverte (13) du flacon, pour fixer les moyens de fixation complémentaires sur les moyens de fixation pour fixer l'adaptateur sur le dispositif d'arrêt, caractérisé en ce qu'un trajet d'écoulement interne (33) s'étend à travers ledit adaptateur, l'extrémité arrière ayant une aiguille creuse (36) dirigée vers l'arrière, munie d'un bout pointu (37) définissant l'extrémité arrière du trajet d'écoulement, et étant adaptée pour percer le dispositif d'arrêt par l'intermédiaire de l'aiguille pour établir une liaison d'écoulement entre le liquide du flacon et le trajet d'écoulement, et l'extrémité avant ayant une buse creuse (39) dirigée vers l'avant définissant l'extrémité avant du trajet d'écoulement, un clapet anti-retour (40) pouvant être ouvert par une sonde de contact mécanique et se fermant de manière automatique situé dans le trajet d'écoulement adjacent à la buse, et un connecteur (45) formant verrou adapté pour être verrouillé de manière libérable sur un connecteur (69) formant verrou complémentaire situé sur un dispositif dispensateur (60) séparé actionné par aspiration ayant une buse creuse complémentaire (66) contenant une sonde de contact mécanique (70) agencée pour établir une liaison d'écoulement avec la buse et pour ouvrir le clapet.

13. Adaptateur selon la revendication 12, dans lequel l'extrémité arrière (32) comporte une tubulure (35) contenant l'aiguille (36), et le bout de l'aiguille (37) est situé dans la tubulure en direction de l'intérieur à partir de l'extrémité arrière.

14. Adaptateur selon la revendication 13, dans lequel les moyens de fixation complémentaires (38) sont situés dans la tubulure.

15. Adaptateur selon la revendication 14, dans lequel les moyens de fixation complémentaires (38) sont des moyens de liaison par filetage.

16. Adaptateur selon l'une quelconque des revendications 12 à 15, dans lequel le connecteur formant verrou est un connecteur formant verrou du type Luer.

17. Adaptateur (30) selon l'une quelconque des revendications 12 à 16, dans lequel l'adaptateur a une forme de manière générale cylindrique, l'aiguille (36) étant située axialement dans celui-ci.

18. Procédé d'utilisation de l'ensemble (1) selon la revendication 10 pour transférer pratiquement sans fuite un volume choisi de liquide médicamenteux (1) à partir de la chambre (14) du flacon via l'adaptateur (30) vers le dispositif (60) séparé actionné par aspiration, comportant les étapes consistant à :
(a) fixer les moyens de fixation complémentaires (38) sur les moyens de fixation (19) et percer le dispositif d'arrêt (15) par l'intermédiaire de l'aiguille (36) pour fixer l'adaptateur sur le dispositif d'arrêt et établir un écoulement entre la chambre du flacon et le trajet d'écoulement (33) de l'adaptateur,
(b) verrouiller le connecteur (69) formant verrou complémentaire sur le connecteur (45) formant verrou pour établir un écoulement entre la buse complémentaire (66) et la buse (38) et ouvrir mécaniquement le clapet (40) par l'intermédiaire de la sonde de contact (70) pour établir un écoulement entre le dispositif et le trajet d'écoulement de l'adaptateur,
(c) transférer un volume choisi du liquide depuis la chambre jusqu'à l'intérieur du dispositif par une opération d'aspiration du dispositif et effectuer de manière simultanée la compensation du déplacement du flacon longitudinalement par rapport au dispositif d'arrêt soumis à la pression atmosphérique extérieure pour réduire le volume de la chambre d'une quantité de manière générale égale à celle du liquide transféré, et
(d) déconnecter le dispositif de l'adaptateur en déverrouillant le connecteur formant verrou complémentaire du connecteur formant verrou ceci s'accompagnant de la fermeture automatique du clapet pour empêcher une fuite à partir de la buse.

19. Procédé selon la revendication 18, dans lequel la chambre (14) remplie de liquide est pratiquement exempte d'espaces d'air d'accompagnement.

20. Procédé selon la revendication 18 ou 19, dans lequel les étapes (b), (c) et (d) sont répétées pour transférer un autre volume choisi de liquide depuis la chambre (14) du flacon vers le dispositif (60).

21. Procédé selon la revendication 18, 19 ou 20, dans lequel le dispositif (60) est une seringue.

22. Ensemble selon l'une quelconque des revendications 1 à 9, dans lequel l'adaptateur (130) est formé d'une première et d'une seconde parties, le clapet (140) est reçu à l'intérieur d'un ensemble (146) qui constitue ladite seconde partie de l'adaptateur, ledit ensemble étant monté de manière amovible sur la première partie de l'adaptateur.

23. Ensemble selon la revendication 22, dans lequel ledit ensemble (146) est maintenu de manière amovible sur ladite première partie de l'adaptateur par un bouchon (74), une ouverture (180) étant formée dans le bouchon, à travers laquelle s'étend la buse creuse (139) dirigée vers l'avant.

24. Ensemble selon la revendication 23, dans lequel le bouchon (174) est encliqueté sur la première partie de l'adaptateur.

25. Ensemble selon la revendication 24, dans lequel le bouchon (174) a un rebord (181) dirigé vers l'extérieur qui s'étend radialement au-delà de la périphérie extérieure du flacon (10) lorsque le flacon et l'adaptateur sont assemblés de manière active, ledit rebord de bouchon ayant une configuration non-circulaire.

26. Adaptateur selon l'une quelconque des revendications 12 à 17, dans lequel l'adaptateur est formé d'une première et d'une seconde parties, le clapet (140) étant reçu à l'intérieur d'un ensemble (146) qui forme ladite seconde partie, ledit ensemble étant monté de manière amovible sur la première partie.

27. Adaptateur (130) selon la revendication 26, dans lequel ladite première partie et ladite seconde partie sont maintenues réunies de manière amovible par un bouchon (174), une ouverture (180) étant formée dans le bouchon, à travers laquelle s'étend la buse creuse (139) dirigée vers l'avant.

28. Adaptateur (130) selon la revendication 27, dans lequel ledit bouchon (174) est encliqueté sur ladite première partie.
